# FASCICULE DE BREVET EUROPEEN

(11) **EP 2 863 870 B1**
(45) Date de publication et mention de la délivrance du brevet: **17.06.2020**
(21) Numéro de dépôt: 13737336.1
(22) Date de dépôt: 20.06.2013
(51) Int. Cl.: A61K 8/25, A61Q 5/00, A61K 8/81, A61K 8/02

(54) **COMPOSITION COSMÉTIQUE DES PARTICULES D'AÉROGEL DE SILICE HYDROPHOBE ET UN POLYMÈRE ÉPAISSISSANT ACRYLIQUE**
KOSMETISCHE ZUSAMMENSETZUNG AUS HYDROPHOBEN SILICA-AEROGELPARTIKELN UND EINEM VERDICKENDEN ACRYLPOLYMER
COSMETIC COMPOSITION OF HYDROPHOBIC SILICA AEROGEL PARTICLES AND AN ACRYLIC THICKENING POLYMER

(30) Priorité: 21.06.2012 FR 1255855; 20.11.2012 US 201261728265 P
(43) Date de publication de la demande: 29.04.2015
(60) Demande divisionnaire: 20167688.9
(73) Titulaire: L'Oréal, 75008 Paris (FR)
(72) Inventeur: KHENNICHE, Samira, F-92110 Clichy (FR); PLOS, Grégory, F-75018 Paris (FR)
(74) Mandataire: L'Oreal
(86) Numéro de dépôt international: PCT/FR2013/051436
(87) Numéro de publication internationale: WO 2013/190238

(56) Documents cités:
- EP-A1- 1 779 840
- EP-A1- 1 779 840
- WO-A1-2013/087926
- WO-A1-2013/087927
- WO-A1-2013/160362
- WO-A2-2012/084780
- WO-A2-2012/084781
- WO-A2-2013/117550
- WO-A2-2013/117550
- DE-A1- 19 506 141
- DE-A1-102007 053 616
- DE-A1-102007 053 616
- US-B1- 6 290 941
- DATABASE GNPD MINTEL; novembre 2006 (2006-11), "Hair Refresher with Jade Blossom", XP002696272, Database accession no. 610899
- DATABASE GNPD [Online] MINTEL; mai 2010 (2010-05), "Dry Dust", XP002696271, Database accession no. 1335285
- TODD OSTERGAARD ET AL: "Next-Generation Rheology Control Brings New Formulating Options for Personal Care", 20120101, 1 février 2012 (2012-02-01), pages 1-6, XP007921836,
- "Product Information VM-2270 Aerogel Fine Particles", , 12 juillet 2007 (2007-07-12), XP055131760, Extrait de l'Internet: URL:http://www.cabot-corp.com/wcm/download /en-us/ae/VM2270 Product Information.pdf [extrait le 2014-07-25]

## Description

La présente invention concerne un procédé de traitement capillaire des matières kératiniques, permettant de retarder le regraissage des cheveux et/ou du cuir chevelu, comprenant l'application d'une composition cosmétique qui comprend l'association de silice hydrophobe et de polymère épaississant particulier.

Les chevelures ont tendance à perdre certaines de leurs qualités sous l'action de facteurs tels que le regraissage naturel, la sueur, l'élimination de squames, la pollution, l'humidité et autres facteurs. L'aspect visuel ou le toucher des cheveux peut ainsi être dégradé. Le regraissage, par exemple dû à la pollution, alourdit les cheveux qui ont alors tendance à se mettre en paquets. Les cheveux peuvent être difficiles à coiffer en plus d'avoir par exemple une brillance grasse ou un toucher ciré désagréable. Pour lutter contre ces désagréments, on peut utiliser des compositions détergentes, par exemple des shampoings, afin d'éliminer les salissures (sébum, sueur, pollution, etc.) ou les pellicules, et détendre les cheveux. La chevelure est ensuite rincée puis séchée. Les shampooings doivent être renouvelés régulièrement, par exemple au bout de quelques jours voire au bout de quelques heures. Toutefois, les shampooings, à base de quantités importantes de tensioactifs, peuvent générer des désagréments tels que des picotements sur le cuir chevelu ou dans les yeux.

Les compositions de shampoing ou de lavage de la peau peuvent également associer aux tensioactifs, des absorbeurs de sébum pour permettre de prolonger dans le temps la perception de propreté des cheveux ou de la peau.

On connait ainsi le produit Stila Hair Refresher (Mintel 610899) qui est une poudre capillaire absorbant les huiles et qui comprend notamment de l'amidon.
On connait également le produit Dry Dust (Mintel 1335285) qui est un produit capillaire comprenant des poudres minérales de type silica silylate qui absorbent l'excès d'humidité du cheveu.

Toutefois, on a constaté que l'efficacité de ces produits est encore insuffisante par rapport aux attentes des consommateurs. En effet, ils ne permettent pas de réduire de manière significative la fréquence de lavage des cheveux notamment, fréquence qui peut différer selon le pays ou même de la région concernée et qui peut aller d'un à deux shampooings par jour à un ou deux shampooings par semaine.
A ce jour, aucune composition cosmétique d'hygiène, notamment capillaire, ne permet de ralentir de manière significative le regraissage, notamment des cheveux.

La présente invention a pour but de proposer un procédé mettant en œuvre des compositions cosmétiques palliant ces inconvénients.
Ces compositions permettent de conserver une perception de cheveux propres pendant un temps plus long qu'avec un shampoing usuel; à titre d'exemple, cette perception de cheveux propres peut être d'une semaine pour les personnes se lavant habituellement les cheveux 2 à 3 fois par semaine, et peut être d'au moins trois jours pour les personnes se lavant habituellement les cheveux tous les jours. Par ailleurs, les compositions selon l'invention permettent d'obtenir des performances de nettoyage au moins identiques à celles d'un shampooing standard, notamment un très bon pouvoir de détergence.

L'invention a donc pour objet un procédé de traitement capillaire des matières kératiniques, comprenant l'application sur lesdites matières d'une composition cosmétique comprenant des particules d'aérogel de silice hydrophobe et au moins un polymère épaississant acrylique choisi parmi les homopolymères d'acide acrylique réticulés, ledit procédé permettant de retarder le regraissage des cheveux et/ou du cuir chevelu.
On a constaté que l'utilisation des compositions selon l'invention permet de réduire de manière significative le regraissage des cheveux et/ou du cuir chevelu, et permet ainsi de réduire la fréquence de lavage.
En outre, la composition selon l'invention permet d'obtenir de bonnes propriétés de conditionnement des cheveux, notamment en terme de douceur, souplesse, lissage et démêlage, tout en ayant une répartition et un étalement sur la chevelure améliorés.

Dans la présente description, l'expression "au moins un" est équivalente à l'expression "un ou plusieurs" et peut y être substituée.
Dans la présente description, l'expression "compris entre" est équivalente à l'expression "allant de " et peut y être substituée.

### Particules d'aérogel de silice hydrophobe

La composition mise en œuvre selon l'invention comprend donc des particules d'aérogel de silice hydrophobe.

Les aérogels sont des matériaux poreux ultra légers, dont les premiers ont été réalisés par Kristler en 1932.
Ils sont généralement synthétisés par un procédé sol-gel en milieu liquide puis séchés par extraction d'un fluide supercritique. Le fluide supercritique le plus communément utilisé est le CO₂ supercritique. Ce type de séchage permet d'éviter la contraction des pores et du matériau. D'autres types de séchage permettent également d'obtenir des matériaux poreux à partir de gel, à savoir par exemple (i) le séchage par cryodessiccation, consistant à solidifier à faible température le gel puis à sublimer le solvant et (ii) le séchage par évaporation. Les matériaux ainsi obtenus sont appelés respectivement cryogels et xérogels. Le procédé sol-gel et les différents séchages sont décrits en détail dans Brinker CJ., and Scherer G.W., Sol-Gel Science: New York: Academic Press, 1990.

Par "silice hydrophobe", on entend toute silice dont la surface est traitée par des agents de silylation, par exemple par des silanes halogénés tels que des alkylchlorosilanes; des siloxanes en particulier des diméthylsiloxanes tels que l'hexaméthyldisiloxane; ou des silazanes, de manière à fonctionnaliser les groupements OH par des groupements silyles Si-Rn, par exemple des groupements triméthylsilyles.

De préférence, les particules d'aérogel de silice hydrophobe susceptibles d'être utilisées dans la présente invention présentent avantageusement une surface spécifique par unité de masse (SM) allant de 500 à 1500 m²/g, de préférence de 600 à 1200 m²/g et mieux de 600 à 800 m²/g.
De préférence, les particules d'aérogel de silice hydrophobe susceptibles d'être utilisées dans la présente invention présentent avantageusement une capacité d'absorption d'huile mesurée au WET POINT allant de 5 à 18 ml/g de particules, de préférence de 6 à 15 ml/g et mieux de 8 à 12 ml/g.
De préférence, les particules d'aérogel de silice hydrophobe susceptibles d'être utilisées dans la présente invention présentent avantageusement une taille, exprimée en diamètre moyen (D[0,5]), inférieure à 1500 µm et de préférence allant de 1 à 30 µm, de préférence de 5 à 25 µm, mieux de 5 à 20 µm et encore mieux de mieux de 5 à 15 µm.
De préférence, les particules d'aérogel de silice hydrophobe susceptibles d'être utilisées dans la présente invention présentent avantageusement une densité tassée p allant de 0,04 g/cm³ à 0,10 g/cm³, de préférence de 0,05 g/cm³ à 0,08 g/cm³.
De préférence, les particules d'aérogel de silice hydrophobe susceptibles d'être utilisées dans la présente invention présentent avantageusement une surface spécifique par unité de volume (SV) allant de 5 à 60 m²/cm³, de préférence de 10 à 50 m²/cm³ et mieux de 15 à 40 m²/cm³.

Selon un mode de réalisation préféré, les particules d'aérogel de silice hydrophobe selon l'invention présentent une surface spécifique par unité de masse (SM) allant de 500 à 1500 m²/g, de préférence de 600 à 1200 m²/g et mieux de 600 à 800 m²/g, et une taille exprimée en diamètre moyen (D[0,5]) allant de 1 à 30 µm et/ou une capacité d'absorption d'huile mesurée au WET POINT allant de 5 à 18 ml/g de particules, de préférence de 6 à 15 ml/g et mieux de 8 à 12 ml/g.

Selon un autre mode de réalisation préféré, les particules d'aérogel de silice hydrophobe utilisées dans la présente invention présentent une surface spécifique par unité de masse (SM) allant de 600 à 800 m²/g et une taille exprimée en diamètre moyen en volume (D[0,5]) allant de 5 à 20 µm, mieux de 5 à 15 µm.

La surface spécifique par unité de masse peut être déterminée par la méthode d'absorption d'azote appelée méthode BET (BRUNAUER - EMMET - TELLER) décrite dans « The journal of the American Chemical Society », vol. 60, page 309, février 1938 et correspondant à la norme internationale ISO 5794/1 (annexe D). La surface spécifique BET correspond à la surface spécifique totale des particules considérées.
La capacité d'absorption mesurée au WET POINT, et notée Wp, correspond à la quantité d'huile qu'il faut additionner à 100 g de particules pour obtenir une pâte homogène. Elle est mesurée selon la méthode dite de Wet Point ou méthode de détermination de prise d'huile de poudre selon le principe décrit dans la norme NF T 30-022. Elle correspond à la quantité d'huile adsorbée sur la surface disponible de la poudre et/ou absorbée par la poudre par mesure du Wet Point, décrite ci-dessous :
On place une quantité m = 2 g de poudre sur une plaque de verre puis on ajoute goutte à goutte l'huile (isononyl isononanoate). Après addition de 4 à 5 gouttes d'huile dans la poudre, on mélange à l'aide d'une spatule et on continue d'ajouter de l'huile jusqu'à la formation de conglomérats d'huile et de poudre. A partir de ce moment, on ajoute l'huile à raison d'une goutte à la fois et on triture ensuite le mélange avec la spatule. On cesse l'addition d'huile lorsque l'on obtient une pâte ferme et lisse. Cette pâte doit se laisser étendre sur la plaque de verre sans craquelures ni formation de grumeaux. On note alors le volume Vs (exprimé en ml) d'huile utilisé.
La prise d'huile (capacité d'absorption d'huile) correspond au rapport Vs / m.
Les tailles des particules d'aérogel selon l'invention peuvent être mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.
Dans le cadre de la présente invention, la densité tassée peut être appréciée selon le protocole suivant, dit protocole de la densité tassée : 40 g de poudre sont versés dans une éprouvette graduée puis l'éprouvette est placée sur un appareil STAV 2003 de chez STAMPF VOLUMETER. L'éprouvette est ensuite soumise à une série de 2500 tassements (cette opération est recommencée jusqu'à ce que la différence de volume entre 2 essais consécutifs soit inférieure à 2 %); puis le volume final Vf de poudre tassée est mesuré directement sur l'éprouvette. La densité tassée est déterminée par le rapport masse(m)/Vf, en l'occurrence 40/Vf (Vf étant exprimé en cm³ et m en g).
La surface spécifique par unité de volume est donnée par la relation : SV = SM x p, où p est la densité tassée exprimée en g/cm³ et SM est la surface spécifique par unité de masse exprimée en m²/g, telles que définies plus haut.

Les particules d'aérogel de silice hydrophobe utilisées selon la présente invention sont de préférence des particules d'aérogel de silice silylée (nom INCI silica silylate).
La préparation de particules d'aérogels de silice hydrophobe modifiées en surface par silylation est décrite plus avant dans le document US 7,470,725.
On utilisera en particulier des particules d'aérogels de silice hydrophobe modifiée en surface par groupements triméthylsilyles.

A titre de d'aérogels de silice hydrophobe utilisables dans l'invention, on peut citer par exemple l'aérogel commercialisé sous la dénomination VM-2260 (nom INCI Silica silylate), par la société Dow Corning, dont les particules présentent une taille moyenne d'environ 1000 microns et une surface spécifique par unité de masse allant de 600 à 800 m²/g.
On peut également citer les aérogels commercialisés par la société Cabot sous les références AEROGEL TLD 201, AEROGEL OGD 201 et AEROGEL TLD 203, ENOVA AEROGEL MT 1100, ENOVA AEROGEL MT 1200.
On utilisera plus particulièrement l'aérogel commercialisé sous la dénomination VM-2270 (nom INCI Silica silylate), par la société Dow Corning, dont les particules présentent une taille moyenne allant de 5 à 15 microns et une surface spécifique par unité de masse allant de 600 à 800 m²/g.

On peut bien évidemment utiliser un mélange de particules d'aérogel de silice hydrophobe.

Les compositions selon l'invention peuvent comprendre les particules d'aérogel de silice hydrophobe en une quantité comprise entre 0,01 et 20% en poids, de préférence entre 0,05 et 10% en poids, préférentiellement entre 0,1 et 5% en poids par rapport au poids total de la composition.

### Polymères épaississants

La composition mise en œuvre selon l'invention comprend également au moins un polymère épaississant acrylique. On peut bien évidemment utiliser un mélange de polymères épaississants acryliques.

Par polymère épaississant, on entend au sens de la présente invention un polymère présentant en solution ou en dispersion à 1% en poids de matière active dans l'eau ou dans l'éthanol, à 25°C, une viscosité supérieure à 0,2 Poise, à un taux de cisaillement de 1 s⁻¹. La viscosité est mesurée avec un viscosimètre HAAKE RS600 de THERMO ELECTRON. Ce viscosimètre est un viscosimètre à contrainte imposée en géométrie cône plan (par exemple de diamètre 60 mm)

Les polymères épaississants acryliques utilisés dans la présente invention sont choisis parmi les homopolymères d'acide acrylique réticulés.

Parmi les homopolymères d'acide acrylique réticulés, on peut citer les produits vendus sous les noms CARBOPOLS 980, 981, 954, 2984 et 5984 par la société GOODRICH ou les produits vendus sous les noms SYNTHALEN M et SYNTHALEN K par la société 3 VSA.

La composition selon l'invention comprend le ou les polymères épaississants acryliques en une quantité de préférence comprise entre 0,01 et 2% en poids, notamment 0,1 à 0,5% en poids, par rapport au poids total de la composition.

### Autres ingrédients

La composition cosmétique selon l'invention comprend généralement un milieu cosmétiquement acceptable, c'est-à-dire un milieu compatible avec les matières kératiniques telles que la peau du visage ou du corps, les lèvres, les cheveux, les cils, les sourcils et les ongles.

Les compositions selon l'invention peuvent se présenter sous toutes les formes galéniques classiquement utilisées, et notamment sous forme d'une solution ou suspension aqueuse, alcoolique ou hydroalcoolique, ou huileuse; d'une solution ou d'une dispersion du type lotion ou sérum; d'une émulsion, notamment de consistance liquide ou semi-liquide, du type H/E, E/H ou multiple; d'une suspension ou émulsion de consistance molle de type crème (H/E) ou (E/H); d'un gel aqueux ou anhydre, ou de toute autre forme cosmétique.
Ces compositions peuvent être conditionnées dans des flacons pompes ou dans des récipients aérosols, afin d'assurer une application de la composition sous forme vaporisée (laque) ou sous forme de mousse. De telles formes de conditionnement sont indiquées, par exemple, lorsqu'on souhaite obtenir un spray ou une mousse, pour le traitement des cheveux. Dans ces cas, la composition comprend de préférence au moins un agent propulseur.

La composition selon l'invention peut être aqueuse ou anhydre. Elle est de préférence aqueuse et comprend alors de l'eau à une concentration allant de préférence de 5 à 98% en poids, notamment de 20 à 95% en poids, mieux de 50 à 90% en poids, par rapport au poids total de la composition
La composition peut également comprendre un ou plusieurs solvants organiques liquides à 25°C, 1 atm., notamment hydrosolubles, tels que les alcools en C1-C7; on peut notamment citer les monoalcools aliphatiques ou aromatiques en C1-C7, les polyols et les éthers de polyols en C3-C7, qui peuvent donc être employés seuls ou en mélange avec de l'eau. Avantageusement, le solvant organique peut être choisi parmi l'éthanol, l'isopropanol, l'alcool benzylique et leurs mélanges.

La composition selon l'invention peut en outre comprendre au moins un ingrédient cosmétique usuel, différent des composés de l'invention, et notamment choisi parmi les propulseurs; les huiles végétales, minérales, animales ou de synthèse; les corps gras solides et notamment les cires, les esters en C8-C40, les acides en C8-C40; les alcools en C8-C40; les tensioactifs non ioniques, les tensioactifs cationiques, les tensioactifs anioniques, les tensioactifs amphotères, les tensioactifs zwittérioniques; les filtres solaires; les agents hydratants; les agents antipelliculaires; les agents antioxydants; les agents chélatants; les agents réducteurs; les bases d'oxydation, les coupleurs, les agents oxydants, les colorants directs; les agents défrisants tels que les thiols et les hydroxydes alcalins; les agents nacrants et opacifiants; les agents plastifiants ou de coalescence; les hydroxyacides; les pigments; les charges; les silicones et en particulier les polydiméthylsiloxanes (PDMS); les polymères conditionneurs ou coiffants; les parfums; les agents d'alcalinisation ou d'acidification; les silanes; les agents de réticulation tels que les polyphénols, les aldéhydes, la DHA. La composition peut bien évidemment comprendre plusieurs ingrédients cosmétiques figurant dans la liste ci-dessus.
Selon leur nature et la destination de la composition, les ingrédients cosmétiques usuels peuvent être présents en des quantités usuelles, aisément déterminables par l'homme du métier, et qui peuvent être comprises, de manière générale, pour chaque ingrédient, entre 0,01 à 80% en poids.

Les huiles peuvent être préférentiellement présentes à raison de 0,01 à 80% en poids, notamment 0,02 à 40% en poids, voire 0,5 à 20% en poids, par rapport au poids total de la composition. Elles peuvent être carbonées. On peut notamment citer les huiles végétales, animales ou minérales, hydrogénées ou non, les huiles synthétiques hydrocarbonées, cycliques ou aliphatiques, linéaires ou ramifiées, saturées ou insaturées, telles que par exemple les polyalpha-oléfines, en particulier les polydécènes et polyisobutènes; les alcools gras liquides tels que l'alcool isostéarylique, l'octyldodécanol, le 2-butyloctanol, le 2-hexyldécanol, le 2-undécylpentadécanol, l'alcool oléique ou l'alcool linoléique; les esters liquides comme le myristate d'isopropyle ou le palmitate d'isopropyle. On peut aussi citer les huiles de silicone, volatiles ou non, organomodifiées ou non, hydrosolubles ou non; les huiles fluorées ou perfluorées; ainsi que leurs mélanges.
La composition peut aussi comprendre un ou plusieurs corps gras solides, et en particulier un ou plusieurs alcools gras, esters gras et/ou acides gras, différents des huiles ci-dessus, ayant 8 à 40 atomes de carbone; ces corps gras solides peuvent préférentiellement être présents à raison de 0,01 à 30% en poids, notamment 0,1 à 20% en poids par rapport au poids total de la composition. On peut notamment citer les alcools gras à chaînes linéaires en C12-C32, notamment en C12-C26, et en particulier l'alcool cétylique, l'alcool stéarylique, l'alcool cétylstéarylique, l'alcool béhénylique. On peut citer également les acides gras à chaînes linéaires ou ramifiées en C16-C40, et notamment l'acide méthyl-18 eicosanoïque, les acides d'huile de coprah ou d'huile de coprah hydrogénée; l'acide stéarique, l'acide laurique, l'acide palmitique et l'acide oléique, l'acide béhénique, et leurs mélanges. De préférence, les acides gras sont non salifiés. On peut encore citer les esters gras à chaînes linéaires, comportant au total entre 8 et 40 atomes de carbone, tels que les myristates, palmitates et stéarates de myristyle, de cétyle, de stéaryle, seuls ou en mélange.
L'homme de métier veillera à choisir les ingrédients entrant dans la composition, ainsi que leurs quantités, de manière à ce qu'ils ne nuisent pas aux propriétés des compositions de la présente invention.

Le pH de la composition, si elle est aqueuse, peut être acide, neutre ou alcalin. De préférence, la composition présente un pH compris entre 2 et 11, notamment 3 à 9, voire 3 à 7.

La composition cosmétique selon l'invention peut se présenter sous la forme d'un produit de soin, de nettoyage et/ou de maquillage de la peau du corps ou du visage, des lèvres, des sourcils, des cils, des ongles et des cheveux, d'un produit solaire ou autobronzant, d'un produit d'hygiène corporelle, d'un produit capillaire, notamment de soin, de nettoyage, de coiffage, de mise en forme, de coloration des cheveux.

Elle trouve notamment une application particulièrement intéressante dans le domaine capillaire, notamment pour le maintien de la coiffure ou la mise en forme des cheveux, ou encore le soin, le traitement cosmétique ou le nettoyage des cheveux. Les compositions capillaires sont de préférence des shampooings, des après-shampooings, des gels de coiffage ou de soin, des lotions ou crèmes de soin, des conditionneurs, des masques, des sérums, des lotions de mise en plis, des lotions pour le brushing, des compositions de fixation et de coiffage telles que les laques ou spray; de lotion restructurante pour cheveux; de lotion ou gel anti-chute, de shampoing antiparasitaire, de lotion ou shampoing antipelliculaire, de shampoing traitant antiséborrhéique. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse.

La composition selon l'invention trouve une application particulièrement intéressante pour le soin, le traitement et/ou le nettoyage des cheveux et/ou du cuir chevelu, par exemple en shampooing, en après shampooing, en post-traitement d'un shampoing et/ou d'un après-shampoing; ou bien entre deux shampoings.
On peut ainsi par exemple appliquer la composition selon l'invention sur le cuir chevelu et/ou les cheveux, directement après un shampooing, sur cheveux humides ou sur cheveux secs, avec ou sans temps de pause, avec ou sans chaleur, ladite application étant suivie ou pas d'un rinçage et d'un séchage, soit à température ambiante soit avec un sèche-cheveu ou encore avec un fer à lisser (250°C) par exemple.
On peut également appliquer la composition selon l'invention sur le cuir chevelu et/ou les cheveux directement après un après-shampooing, sur cheveux humides ou sur cheveux secs, avec ou sans temps de pause, avec ou sans chaleur, ladite application étant suivie ou pas d'un rinçage et d'un séchage, soit à température ambiante soit avec un sèche-cheveu ou encore avec un fer à lisser (250°C) par exemple.
On peut encore appliquer la composition selon l'invention sur le cuir chevelu et/ou les cheveux entre deux shampooings, sur cheveux humides ou sur cheveux secs.

La composition cosmétique peut être rincée ou non après avoir été appliquée sur les matières kératiniques (cheveux et/ou cuir chevelu).

Le procédé selon l'invention est en particulier un procédé de traitement capillaire, pour le soin, le traitement cosmétique et/ou le nettoyage des cheveux et/ou du cuir chevelu, permettant de retarder voire d'éviter le regraissage des cheveux et/ou du cuir chevelu, et ainsi d'espacer les shampoings.

L'invention est illustrée plus en détails dans les exemples suivants.

### Exemple 1

On prépare la composition de soin capillaire suivante (% en poids) :

| | Composition |
|---|---|
| SILICA SILYLATE (nom INCI) | 0,5% |
| Polymère acrylique (Carbopol 980) | 0,3% |
| Eau | Qsp 100% |

Le SILICA SILYLATE employé est le produit commercialisé sous le nom DOW CORNING VM-2270 AEROGEL FINE PARTICLES par Dow Corning.

La composition se présente sous forme d'une dispersion de pH égal à 3,5 et est facile à appliquer sur les cheveux et le cuir chevelu; elle permet de réduire le regraissage des cheveux.

## Revendications

1. Procédé de traitement capillaire des matières kératiniques, comprenant l'application sur lesdites matières d'une composition cosmétique comprenant des particules d'aérogel de silice hydrophobe et au moins un polymère épaississant acrylique choisi parmi les homopolymères d'acide acrylique réticulés,
ledit procédé permettant de retarder le regraissage des cheveux et/ou du cuir chevelu.

2. Procédé selon la revendication 1, dans lequel les particules d'aérogel de silice hydrophobe présentent une surface spécifique par unité de masse (SM) allant de 500 à 1500 m²/g, de préférence de 600 à 1200 m²/g et mieux de 600 à 800 m²/g,

3. Procédé selon l'une quelconque des revendications précédentes, dans lequel les particules d'aérogel de silice hydrophobe présentent une capacité d'absorption d'huile mesurée au WET POINT allant de 5 à 18 ml/g de particules, de préférence de 6 à 15 ml/g et mieux de 8 à 12 ml/g.

4. Procédé selon l'une quelconque des revendications précédentes, dans lequel les particules d'aérogel de silice hydrophobe présentent une taille, exprimée en diamètre moyen (D[0,5]), inférieure à 1500 µm et de préférence allant de 1 à 30 µm, de préférence de 5 à 25 µm, mieux de 5 à 20 µm et encore mieux de mieux de 5 à 15 µm.

5. Procédé selon l'une quelconque des revendications précédentes, dans lequel les particules d'aérogel de silice hydrophobe présentent une densité tassée ρ allant de 0,04 g/cm³ à 0,10 g/cm³, de préférence de 0,05 g/cm³ à 0,08 g/cm³.

6. Procédé selon l'une quelconque des revendications précédentes, dans lequel les particules d'aérogel de silice hydrophobe présentent une surface spécifique par unité de volume (SV) allant de 5 à 60 m²/cm³, de préférence de 10 à 50 m²/cm³ et mieux de 15 à 40 m²/cm³.

7. Procédé selon l'une quelconque des revendications précédentes, dans lequel les particules d'aérogel de silice hydrophobe sont des particules d'aérogel de silice silylée et notamment des particules d'aérogels de silice hydrophobe modifiée en surface par groupements triméthylsilyles.

8. Procédé selon l'une quelconque des revendications précédentes, dans lequel les particules d'aérogel de silice hydrophobe sont présentes en une quantité comprise entre 0,01 et 20% en poids, de préférence entre 0,05 et 10% en poids, préférentiellement entre 0,1 et 5% en poids par rapport au poids total de la composition.

9. Procédé selon l'une quelconque des revendications précédentes, dans lequel la composition comprend de l'eau à une concentration allant de 5 à 98% en poids, notamment de 20 à 95% en poids, mieux de 50 à 90% en poids, par rapport au poids total de la composition.

10. Procédé selon l'une quelconque des revendications précédentes, permettant d'éviter le regraissage des cheveux et/ou du cuir chevelu.

## Patentansprüche

1. Haarbehandlungsverfahren für Keratinmaterialien, bei dem man auf die Materialien eine kosmetische Zusammensetzung aufbringt, die Aerogelteilchen aus hydrophobem Siliciumdioxid und mindestens ein verdickendes Acrylpolymer, das aus vernetzten acrylsäure-Homopolymeren ausgewählt ist, umfasst, wobei das Verfahren eine Verzögerung der Rückfettung der Haare und/oder der Kopfhaut ermöglicht.

2. Verfahren nach Anspruch 1, wobei die Aerogelteilchen aus hydrophobem Siliciumdioxid eine spezifische Oberfläche pro Masseneinheit (SM) im Bereich von 500 bis 1500 m²/g, bevorzugt von 600 bis 1200 m²/g und noch besser von 600 bis 800 m²/g aufweisen.

3. Verfahren nach einem der vorhergehenden Ansprüche, wobei die Aerogelteilchen aus hydrophobem Siliciumdioxid eine am WET POINT gemessene Ölabsorptionskapazität im Bereich von 5 bis 18 ml/g Teilchen, bevorzugt von 6 bis 15 ml/g und noch besser von 8 bis 12 ml/g aufweisen.

4. Verfahren nach einem der vorhergehenden Ansprüche, wobei die Aerogelteilchen aus hydrophobem Siliciumdioxid eine als mittlerer Durchmesser (D[0,5]) ausgedrückte Größe von weniger als 1500 µm und bevorzugt im Bereich von 1 bis 30 µm, bevorzugt von 5 bis 25 µm, noch besser von 5 bis 20 µm und sogar noch besser von 5 bis 15 µm aufweisen.

5. Verfahren nach einem der vorhergehenden Ansprüche, wobei die Aerogelteilchen aus hydrophobem Siliciumdioxid eine Klopfdichte ρ im Bereich von 0,04 g/cm³ bis 0,10 g/cm³ und bevorzugt von 0,05 g/cm³ bis 0,08 g/cm³ aufweisen.

6. Verfahren nach einem der vorhergehenden Ansprüche, wobei die Aerogelteilchen aus hydrophobem Siliciumdioxid eine eine spezifische Oberfläche pro Volumeneinheit (SV) im Bereich von 5 bis 60 m²/cm³, bevorzugt von 10 bis 50 m²/m³ und noch besser von 15 bis 40 m²/cm³ aufweisen.

7. Verfahren nach einem der vorhergehenden Ansprüche, wobei es sich bei den Aerogelteilchen aus hydrophobem Siliciumdioxid um Aerogelteilchen aus silyliertem Siliciumdioxid und insbesondere Aerogelteilchen aus an der Oberfläche mit Trimethylsilylgruppen modifiziertem Siliciumdioxid handelt.

8. Verfahren nach einem der vorhergehenden Ansprüche, wobei die Aerogelteilchen aus hydrophobem Siliciumdioxid in einer Menge zwischen 0,01 und 20 Gew.-%, bevorzugt zwischen 0,05 und 10 Gew.-%, vorzugsweise zwischen 0,1 und 5 Gew.-%, bezogen auf das Gesamtgewicht der Zusammensetzung, vorliegen.

9. Verfahren nach einem der vorhergehenden Ansprüche, wobei die Zusammensetzung Wasser in einer Konzentration im Bereich von 5 bis 98 Gew.-%, insbesondere von 20 bis 95 Gew.-%, noch besser von 50 bis 90 Gew.-%, bezogen auf das Gesamtgewicht der Zusammensetzung, umfasst.

10. Verfahren nach einem der vorhergehenden Ansprüche, dass eine Vermeidung der Rückfettung der Haare und/oder der Kopfhaut ermöglicht.

## Claims

1. Method for a hair treatment of keratin materials, comprising the application, to the said materials, of a cosmetic composition comprising hydrophobic silica aerogel particles and at least one acrylic thickening polymer chosen from crosslinked acrylic acid homopolymers,
the said method making it possible to delay the regreasing of the hair and/or of the scalp.

2. Method according to Claim 1, in which the hydrophobic silica aerogel particles exhibit a specific surface per unit of mass (S_{M}) ranging from 500 to 1500 m²/g, preferably from 600 to 1200 m²/g and better still from 600 to 800 m²/g.

3. Method according to either one of the preceding claims, in which the hydrophobic silica aerogel particles exhibit an oil absorption capacity, measured at the wet point, ranging from 5 to 18 ml/g of particles, preferably from 6 to 15 ml/g and better still from 8 to 12 ml/g.

4. Method according to any one of the preceding claims, in which the hydrophobic silica aerogel particles exhibit a size, expressed as mean diameter (D[0.5]), of less than 1500 µm and preferably ranging from 1 to 30 µm, preferably from 5 to 25 µm, better still from 5 to 20 µm and even better still from 5 to 15 µm.

5. Method according to any one of the preceding claims, in which the hydrophobic silica aerogel particles exhibit a tapped density ρ ranging from 0.04 g/cm³ to 0.10 g/cm³, preferably from 0.05 g/cm³ to 0.08 g/cm³.

6. Method according to any one of the preceding claims, in which the hydrophobic silica aerogel particles exhibit a specific surface per unit of volume (S_{V}) ranging from 5 to 60 m²/cm³, preferably from 10 to 50 m²/cm³ and better still from 15 to 40 m²/cm³.

7. Method according to any one of the preceding claims, in which the hydrophobic silica aerogel particles are silylated silica aerogel particles and in particular aerogel particles of hydrophobic silica modified at the surface by trimethylsilyl groups.

8. Method according to any one of the preceding claims, in which the hydrophobic silica aerogel particles are present in an amount of between 0.01% and 20% by weight, preferably between 0.05% and 10% by weight, preferentially between 0.1% and 5% by weight, with respect to the total weight of the composition.

9. Method according to any one of the preceding claims, in which the composition comprises water at a concentration ranging from 5% to 98% by weight, in particular from 20% to 95% by weight, better still from 50% to 90% by weight, with respect to the total weight of the composition.

10. Method according to any one of the preceding claims, which makes it possible to prevent the regreasing of the hair and/or of the scalp.
